# EUROPEAN PATENT SPECIFICATION

(11) **EP 2 486 403 B1**
(45) Date of publication and mention of the grant of the patent: **26.06.2013**
(21) Application number: 09737469.8
(22) Date of filing: 09.10.2009
(51) Int. Cl.: G01N 33/497

(54) **BREATH SENSOR**
ATEMSENSOR
DÉTECTEUR DE RESPIRATION

(43) Date of publication of application: 15.08.2012
(73) Proprietor: Autoliv Development AB, 447 83 Vårgårda (SE)
(72) Inventor: GRAAN, Hans, S-59172 Motala (SE)
(74) Representative: Forrester, Simon Joseph
(86) International application number: PCT/GB2009/051349
(87) International publication number: WO 2011/042679

(56) References cited:
- KR-B1- 100 846 062
- US-A- 5 291 898
- US-A- 6 040 362
- US-A1- 2004 181 346

## Description

### Description of Invention

THIS INVENTION relates to a sensor, and in particular concerns a sensor for determining, through analysis of the breath of a subject, whether the subject is likely to have taken or consumed one or more substances, such as alcohol, which might impair the subject's ability to drive a motor vehicle.

In many countries it is illegal to carry out certain activities, such as driving a motor vehicle or operating machinery, whilst under the influence of intoxicating substances such as alcohol. One convenient method of determining whether a subject is or maybe over the prescribed legal limit for any such substance is to analyse the exhaled breath of the subject. Many different types of breath analyser have been developed for this purpose, but most types of analyser comprise a substantially enclosed chamber into which a subject exhaled breath is directed and stored. A beam of light, for instance infrared light, is then directed through the sample chamber, and is absorbed by a detector. The presence of certain traces of substances within the exhaled breath can be analysed through investigating the regions of the light spectrum which are absorbed as the light passes through the exhaled breath.

It is often desirable for the analyser to be as small as possible, for instance if they are to be used in hand-held devices as used by the police, or for sensors which are to be installed in a vehicle dashboard to determine if an occupant or the driver maybe over the prescribed legal limit for alcohol/drugs. For this reason sensors are often arranged so that the beam of light is reflected multiple times within the chamber, so that the beam of light passes through the sample of exhaled breath several times. This can give the beam of light a relatively long effective path length through the exhaled breath, without correspondingly increasing the size of the sample chamber.

The internal surfaces of sample chambers are therefore often made to be reflective. However, exhaled breath is often at an elevated temperature when compared to the ambient air, and as freshly-exhaled breath is directed into a sample chamber condensation can often form on the reflective surfaces. This will reduce the reflectivity of the surfaces, so that reduced intensity of light reaches the detector. Clearly, this will reduce the consistency and reliability of the detector.

To overcome this difficulty, it has previously been proposed to heat the internal reflective surfaces of a sample chamber to at least around 34°C, to avoid the formation of condensation. To achieve this, a wire may be wrapped around the exterior of the sample chamber, and a current passed through the wire so that the temperature of the wire rises through resistive heating, thus heating the sample chamber and the reflective surfaces. An example of such an arrangement is shown in US5222389.

It is an object of the present invention to provide an improved sensor/analyser of this type. sensor/analyser of this type.

Accordingly, one aspect of the present invention provides a sensor according to claim 1 for analysing exhaled breath, the sensor comprising: a body comprising a lid and a base, the lid and base being formed from one or more materials having low thermal conductivity, the lid and base being fitted together to define an enclosure which is substantially enclosed; thin reflective layers or coatings formed on respective inner surfaces of the lid and base, the layers or coatings on the lid and base being in conductive contact with one another; a substrate on which the body is mounted, the substrate defining at least one region which is conductive to heat or current; a source of heat or current provided outside the enclosure and connected to provide current or heat to the at least one region of the substrate; a conductor protruding from the body to contact the at least one region of the substrate, to transmit heat or current from the at least one region to the reflective layers or coatings formed on the lid and the base; and a connection between the conductor and the at least one region of the substrate, the connection allowing conduction of heat or current and also allowing for variations in distance between the components of the body and the substrate arising from thermal expansion.

Advantageously, two regions which are conductive to heat or current are provided on the substrate, and two conductors protrude from the body to contact the respective regions of the substrate, to transmit heat or current from the regions to the reflective layers or coatings formed on the lid and the base.

Preferably, the connection comprises one or more quantities of flexible conductive material.

Conveniently, the flexible conductive material is formed from a non-conductive material having a plurality of grains or fibres of a conductive material embedded therein or coated on a surface thereof.

Advantageously, the connection comprises one or more strips of the flexible conductive material.

Preferably, the flexible conductive material comprises a polymeric material having a plurality of grains or fibres of a metal embedded therein or coated on a surface thereof.

Conveniently, the conductor is provided on an extension of the lid which protrudes towards the substrate.

Advantageously, the sensor may further comprising a source and a detector, the source emitting radiation which is reflected from the reflective layers or coatings formed on the inner surfaces of the lid and base before being absorbed by a detector.

The lid and base are formed from one or more materials having low electrical conductivity.

In order that the present invention may be more readily understood, embodiments thereof will now be described, by way of example, in which:
Figure 1 shows a base of a sensor embodying the present invention;
Figure 2 shows a lid of a sensor embodying the present invention; and

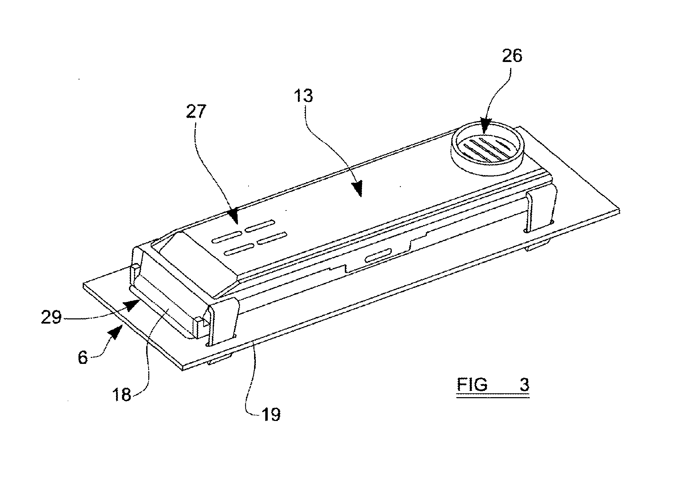

Figure 3 shows the base and lid of figures 1 and 2 assembled to form a sensor.

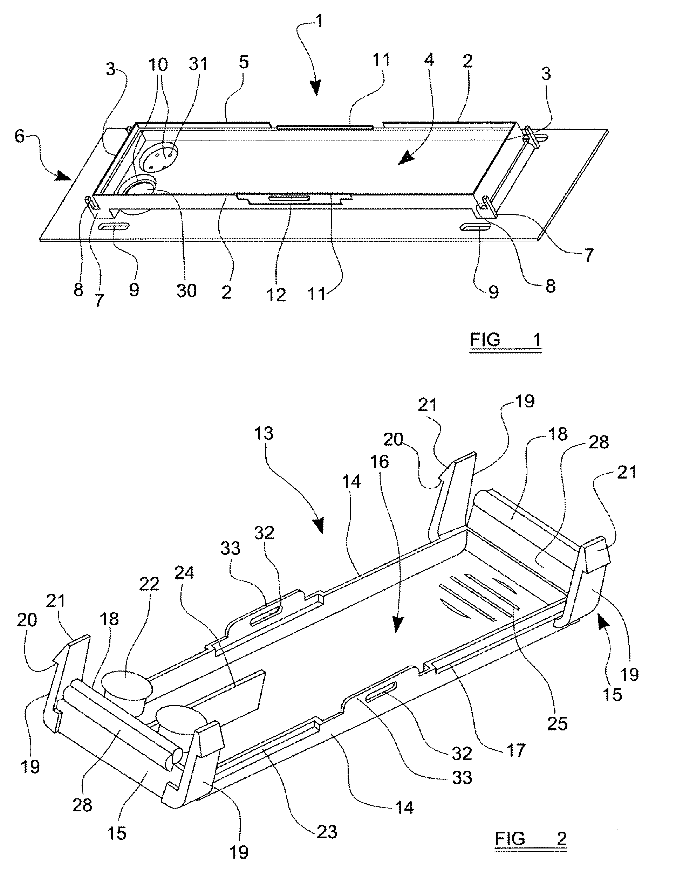

Referring firstly to figure 1, a base 1 of a sensor is shown. The base 1 is generally rectangular in shape, having opposing longer edges 2 and opposing shorter edges 3. The base 1 takes the form of a generally flat, planar bottom surface 4 surrounded by short upstanding walls 5 on all sides.

The base 1 is preferably provided with attachment means to allow the base 1 to be attached to a PCB 6. In the example shown in figure 1, clips 7 are provided at each of the four corners of the base 1. Each clip 7 protrudes substantially directly away from the plane of the bottom surface 4, extending away from the bottom surface 4 in the opposite direction to the direction in which the upstanding walls 5 extend. Each clip terminates at its distal end in an outwardly-flared detent 8.

In an embodiment, the PCB 6 to which the base 1 is to be attached is provided with four elongate slots 9, which are spaced apart with spacing corresponding to that of the clips 7. Each slot 9 has a width similar to the width of each clip 7, and a length which is slightly less than the distance between a back surface of the clip 7 (i.e. the surface of the clip 7 furthest from the detent 8) and the tip of the outwardly-flared detent 8. It will therefore be understood that, as the base 1 is pressed onto the PCB 6, one clip 7 will enter each of the slots 9, and will be able to fit through the slot 9 as the detent 8 will be deflected inwardly. When the detent 8 has passed through the slot 9, the detent 8 will spring outwardly, effectively locking the base 1 in place with respect to the PCB 6.

It will be understood that there are, of course, many other ways in which the base 1 may be attached to a PCB or other circuit board.

A pair of apertures 10 is provided at a first end of the bottom surface 4 of the base 1. These apertures 10 are aligned with corresponding apertures (not shown) in the PCB 6, allowing parts of a source arrangement and a sensing arrangement respectively to protrude through the apertures 10, as will be described in more detail below.

Around mid-sections of the longer sides 2 of the base 1, the upstanding walls 5 describe thinned portions 11, with each thinned portion 11 having a protruding ridge 12 formed on an outer surface thereof.

The base 1 is formed from one or more materials which has a low thermal conductivity and preferably also a high thermal capacity, for instance a plastics material.

A thin reflective coating or surface is formed on the bottom surface 4 of the base 1. The coating or surface is conductive to heat and/or electricity, and is preferably formed from a metallic substance. In preferred embodiments of the invention, the coating or surface is formed from gold or a gold alloy.

In an embodiment, the layer or coating extends continuously to cover the internal surfaces of the upstanding walls 5 at the shorter edges 3 of the base 1.

Turning to figure 2, a lid 13 is shown. The lid 13 takes a generally rectangular form, having opposing longer edges 14 and shorter edges 15, and the dimensions of the lid 13 correspond generally to the dimensions of the base 1.

The lid 13 takes a similar form to the base 1, having a generally flat, planar inner surface 16 and short upstanding walls 17 formed around all sides of the inner surface 16. In addition to the upstanding walls 17, the shorter edges 15 are provided with respective outwardly-flared protrusions 28, which extend away from the plane of the inner surface 16, over the outside of the upstanding walls 17, by a greater amount than the upstanding walls 17, and are also inclined outwardly. The outwardly-flared protrusions 28 are preferably substantially as wide as the shorter edges 15 of the lid 13, and have respective strips 18 of a flexible material provided on or near the free ends thereof. In preferred embodiments the flexible material is a polymer, and even more preferably is an elastomeric material. The flexible material has a plurality of filaments, specks or grains of a conductive material embedded therein, so that the flexible material is conductive to heat and/or electric current.

Legs 19 protrude from each corner of the lid 13, in a direction substantially directly away from the plane of the inner surface 16 thereof, in the same direction as the upstanding walls 17. Each leg 19 is able to deflect inwardly, and at its distal end, on an outer surface, each leg 19 terminates in a tapered surface 21, which forms a point at the distal end and flares outwardly, terminating in a shoulder 20 which is formed on the outer surface of the leg 19 and faces towards the plane of the inner surface 16.

At a first end of the inner surface 16, an infrared (IR) source 22 is provided near the upstanding wall 17 on one side, and an IR receiver 23 is provided near the upstanding wall 17 on the other side. The IR source 22 and IR receiver 23 are separated by a baffle 24 which protrudes directly out of the inner surface 16, substantially perpendicular thereto, from the upstanding walls 5 at the first end around halfway to the central point of the inner surface 16.

At a second end of the lid 13, opposite from the first end, an inlet grille 25 is formed from a plurality of slots formed through the inner surface 16. Near the first end, an outlet grille 27 is formed from a series of further slots.

At around midway points of the longer edges 14, the upstanding walls 17 are provided with thinned portions 33, each of which includes an elongate slot 28 which is substantially parallel with the plane of the inner surface 16, and is of around the same size as the protrusion 12 that protrudes from each of the thinned portions 11 formed in the upstanding walls 5 of the base 1.

The inner surface 16 is provided with a thin conductive, reflective coating or layer, in the same manner as the bottom surface 4 of the base 1. In embodiments of the invention, the reflective layer or coating extends continuously to cover the inner surfaces of the upstanding walls 17 formed at the shorter edges 15 of the lid 13. A conductive arrangement (not shown) is also provided to provide an electrically and/or thermally conductive path from each of the strips 18 of flexible material to the coating or layer formed on the inner surface 16 of the lid 13. For instance, one or more wires or regions of conductive material may be formed within each protrusion 28, or on an inner surface thereof, to provide this conductive arrangement.

To assemble the sensor, the lid 13 is placed over the base 1, so that the legs 19 of the lid 13 pass through further slots 26 formed in the PCB 6. It will be understood that as the legs 19 pass through the further slots 26 they will be deflected inwardly, and will then spring outward so that the shoulder 20 in each leg 21 is abutted against the underside of the PCB 6, thus preventing the lid 13 from being pulled upward and removed from the PCB.

As this occurs, the thinned portions 11,33 of the upstanding walls 5,14 on the base 1 and lid 13 will slide over one another, and the projections 12 in the thinned portions 11 of the base 1 will slot into the slots 28 formed in the thinned portions 33 of the lid 13, further securing the lid 13 and base 1 to one another.

The coatings or layers formed on the bottom surface 4 of the base 1, and on the inner surface 16 of the lid 13, will come into conductive contact with one another as the sensor is assembled. If the coatings or layers formed on the lid 13 and base 1 do not contact each other directly when the sensor is assembled (for instance, if the coatings or layers do not extend to cover the inner surfaces of the updating walls 5,17), then a conductive arrangement may be provided to allow this conductive contact, such as one or more wires or leads.

It will be appreciated that the base 1 and lid 13, when fitted together in this way, will produce a substantially enclosed interior space, which functions as a sample chamber during use of the sensor.

Use of the sensor will now be described.

The inner surfaces 4,16 of the base 1 and lid 13 will act as reflective surfaces during use of the sensor. It is therefore desirable, as discussed above, for these surfaces 4,16 to be heated, to avoid the formation of condensation on the surfaces 4,16 when they become exposed to exhaled breath. A heating arrangement is therefore provided to heat the surfaces 4,16.

The heating arrangement comprises a source of heat or current, which is provided outside the enclosure formed by the base 1 and lid 13. In one embodiment, a source of current (not shown) is provided, connected to conductive tracks on the PCB 6. The current source may be provided and mounted on the PCB 6, or indeed located away from the PCB 6. The current source is connected to one conductive region 29 on the PCB 6 (shown in figure 1) which is formed adjacent to one of the shorter edges 3 of the base 1, and to a second conductive region which is formed adjacent the other short edge 3 of the base 1. In preferred embodiments, one or both of the conductive regions 29 are elongate, parallel with the shorter edges 3, and formed to be approximately the same length as each of the shorter edges 3.

It will be appreciated that, when the lid 13 is secured to the PCB 6, the strips 18 of flexible material will come into electrical contact with the conductive regions 29. Components of the sensor will therefore form a conductive connection between the conductive regions 29, as electricity will be able to flow from one conductive region 29, through one of the strips 18 of flexible material to the conductive coatings formed on the main surface 4 of the base 1 and the inner surface 16 of the base 13. Electricity may then flow through the other one of the strips 18 of flexible material to the second conductive region.

It will therefore be understood that current may flow from the current source through the first conductive region 29, through the conductive coatings of the bottom surface 4 and inner surface 16, heating these surfaces through resistive heating, and to the second conductive region.

In automotive applications it may be expected that the temperature to which the sensor is exposed may vary between -40°C and + 85°C. A significant amount of thermal expansion/contraction is therefore to be expected. However, due to the presence of the strips 18 of flexible material, a reliable electric connection may be maintained between the strips 18 and the conductive regions 29 on the PCB 6, even if the components of the sensor do expand and contract thermally.

A source of heat is provided, for instance a resistive heating coil, which transmits heat to the conductive regions 29 on the PCB 6. This heat will be conducted through these strips 18 of elastomeric material to the main surface 4 of the base 1 and the inner surface 16 of the lid 13, thus causing these surfaces 4,16 to heat up.

To use the sensor a subject is invited to exhale into the inlet grille 26. As this occurs, the enclosure formed within the sensor will partially fill with exhaled breath, and some of the air that was formerly inside the sensor will be driven out of the enclosure through the outlet grille 27.

The IR source 22, which is connected to a source connection 30 that protrudes through one of the apertures 10 formed through the main surface 4 of the base 1, emits infrared light. This light is prevented from being transmitted directly to the IR sensor 23 by the baffle 24. The infrared light therefore travels across the length of the sensor and is reflected from the far wall (i.e. the wall formed at the short end of the sensor opposite the short end of which the IR source 22 and sensor 23 are provided), and is reflected to the IR sensor 23 (which is connected to a sensor connection 31 that protrudes through the other of the apertures 10 formed through the base 1). As this occurs, the infrared radiation will be reflected several times from the internal surfaces of the enclosure.

As is known in the art, the light received at the infrared sensor 23 will allow determinations to be made as to the concentration of volatile substances, such as alcohol, in the exhaled breath.

It will be appreciated that embodiments of the present invention provide a robust and practical sensor, which will perform well in significant temperature variations.

Although strips of flexible material are formed on the protrusions in the above description, other configurations of flexible material may be provided, for instance spaced apart balls or blobs. However, providing elongate strips allows relatively large contact areas without unduly increasing the size of the sensor.

When used in this specification and claims, the terms "comprises" and "comprising" and variations thereof mean that the specified features, steps or integers are included. The terms are not to be interpreted to exclude the presence of other features, steps or components.

## Claims

1. A sensor for analysing exhaled breath, the sensor comprising:
a body comprising a lid (13) and a base (1), the lid (13) and base (1) being formed from one or more materials having low thermal conductivity, the lid (13) and base (1) being fitted together to define an enclosure which is substantially enclosed;
thin reflective layers or coatings formed on respective inner surfaces (16, 4) of the lid (13) and base (1), the layers or coatings on the lid (13) and base (1) being in conductive contact with one another;
a substrate (6) on which the body is mounted, the substrate (6) defining at least one region which is conductive to heat or current;
a source of heat or current provided outside the enclosure and connected to provide current or heat to the at least one region of the substrate (6);
a conductor (18) protruding from the body to contact the at least one region (29) of the substrate (6), to transmit heat or current from the at least one region to the reflective layers or coatings formed on the lid (13) and the base (1); and
a connection between the conductor (18) and the at least one region (29) of the substrate (6), the connection allowing conduction of heat or current and also allowing for variations in distance between the components of the body and the substrate (6) arising from thermal expansion.

2. A sensor according to any preceding claim, wherein two regions (29) which are conductive to heat or current are provided on the substrate (6), and two conductors (18) protrude from the body to contact the respective regions (29) of the substrate (6), to transmit heat or current from the regions (29) to the reflective layers or coatings formed on the lid (13) and the base (1).

3. A sensor according to claim 1 or 2, wherein the connection comprises one or more quantities of flexible conductive material.

4. A sensor according to claim 3, wherein the flexible conductive material is formed from a non-conductive material having a plurality of grains or fibres of a conductive material embedded therein or coated on a surface thereof.

5. A sensor according to claim 3 or 4, wherein the connection comprises one or more strips (18) of the flexible conductive material.

6. A sensor according to any one of claims 3 to 5, wherein the flexible conductive material comprises a polymeric material having a plurality of grains or fibres of a metal embedded therein or coated on a surface thereof.

7. A sensor according to any preceding claim wherein the conductor (18) is provided on an extension (28) of the lid (13) which protrudes towards the substrate (6).

8. A sensor according to any preceding claim further comprising a source and a detector, the source emitting radiation which is reflected from the reflective layers or coatings formed on the inner surfaces (16, 4) of the lid (13) and base (1) before being absorbed by a detector.

9. A sensor according to any preceding claim, wherein the lid (13) and base (1) are formed from one or more materials having low electrical conductivity.

## Patentansprüche

1. Sensor zum Analysieren von ausgeatmeter Luft, wobei der Sensor Folgendes umfasst:
einen Körper, der einen Deckel (13) und ein Unterteil (1) umfasst, wobei der Deckel (13) und das Unterteil (1) aus einem oder mehreren Materialien mit einer geringen Wärmeleitfähigkeit geformt sind, wobei der Deckel (13) und das Unterteil (1) zusammengesetzt sind, um ein Gehäuse zu definieren, das im Wesentlichen geschlossen ist;
dünne reflektierende Lagen oder Schichten, die auf jeweiligen Innenflächen (16, 4) des Deckels (13) und des Unterteils (1) ausgebildet sind, wobei die Lagen oder Schichten auf dem Deckel (13) und dem Unterteil (1) miteinander in leitfähigem Kontakt stehen;
einen Träger (6), auf dem der Körper montiert ist, wobei der Träger (6) mindestens einen Bereich definiert, der wärmeleitfähig oder elektrisch leitfähig ist;
eine Wärme- oder Stromquelle, die außerhalb des Gehäuses vorgesehen ist und angeschlossen ist, um den mindestens einen Bereich des Trägers (6) mit Strom oder Wärme zu versorgen;
einen Leiter (18), der aus dem Körper ragt, um den mindestens einen Bereich (29) des Trägers (6) zu berühren, um Wärme oder Strom von dem mindestens einen Bereich auf die reflektierenden Lagen oder Schichten, die auf dem Deckel (13) und dem Unterteil (1) ausgebildet sind, zu übertragen; und
eine Verbindung zwischen dem Leiter (18) und dem mindestens einen Bereich (29) des Trägers (6), wobei die Verbindung das Leiten von Wärme oder Strom ermöglicht und auch Veränderungen des Abstands zwischen den Bestandteilen des Körpers und dem Träger (6) ermöglicht, die von der Wärmeausdehnung herrühren.

2. Sensor nach einem vorhergehenden Anspruch, wobei zwei Bereiche (29), die wärmeleitfähig oder elektrisch leitfähig sind, auf dem Träger (6) vorgesehen sind, und zwei Leiter (18) aus dem Körper ragen, um die jeweiligen Bereiche (29) des Trägers (6) zu berühren, um Wärme oder Strom von den Bereichen (29) auf die reflektierenden Lagen oder Schichten, die auf dem Deckel (13) und dem Unterteil (1) ausgebildet sind, zu übertragen.

3. Sensor nach Anspruch 1 oder 2, wobei die Verbindung eine oder mehrere Mengen flexiblen leitfähigen Materials umfasst.

4. Sensor nach Anspruch 3, wobei das flexible leitfähige Material aus einem nicht leitfähigen Material geformt ist, das eine Vielzahl von Körnern oder Fasern aus einem leitfähigen Material aufweist, die darin eingebettet oder auf eine Fläche davon aufgebracht sind.

5. Sensor nach Anspruch 3 oder 4, wobei die Verbindung einen oder mehrere Streifen (18) des flexiblen leitfähigen Materials umfasst.

6. Sensor nach einem der Ansprüche 3 bis 5, wobei das flexible leitfähige Material einen Polymerwerkstoff umfasst, der eine Vielzahl von Körnern oder Fasern aus einem Metall aufweist, die darin eingebettet oder auf eine Fläche davon aufgebracht sind.

7. Sensor nach einem vorhergehenden Anspruch, wobei der Leiter (18) an einer Erweiterung (28) des Deckels (13) vorgesehen ist, die in Richtung des Trägers (6) ragt.

8. Sensor nach einem vorhergehenden Anspruch, der ferner eine Quelle und einen Detektor umfasst, wobei die Quelle Strahlung aussendet, die von den reflektierenden Lagen oder Schichten reflektiert wird, die auf den Innenflächen (16, 4) des Deckels (13) und des Unterteils (1) ausgebildet sind, bevor sie von einem Detektor absorbiert wird.

9. Sensor nach einem vorhergehenden Anspruch, wobei der Deckel (13) und das Unterteil (1) aus einem oder mehreren Materialien mit einer geringen elektrischen Leitfähigkeit gebildet sind.

## Revendications

1. Détecteur pour analyser de l'air expiré, le détecteur comprenant :
un corps comprenant un couvercle (13) et une base (1), le couvercle (13) et la base (1) étant formés à partir d'un ou de plusieurs matériaux présentant une faible conductibilité thermique, le couvercle (13) et la base (1) étant assemblés pour définir un boîtier qui est essentiellement clos;
des couches ou revêtements fins réfléchissants formés sur des surfaces intérieures respectives (16, 4) du couvercle (13) et de la base (1), les couches ou revêtements sur le couvercle (13) et la base (1) étant en contact conducteur les uns avec les autres ;
un substrat (6) sur lequel le corps est monté, le substrat (6) définissant au moins une zone qui est conductrice de chaleur ou de courant ;
une source de chaleur ou de courant prévue à l'extérieur du boîtier et connectée pour fournir du courant ou de la chaleur à l'au moins une zone du substrat (6) ;
un conducteur (18) dépassant du corps pour toucher l'au moins une zone (29) du substrat (6), pour transmettre de la chaleur ou du courant de l'au moins une zone aux couches ou revêtements réfléchissants formés sur le couvercle (13) et la base (1); et
une connexion entre le conducteur (18) et l'au moins une zone (29) du substrat (6), la connexion permettant la conduction de chaleur ou de courant et permettant également des variations de distance entre les composants du corps et le substrat (6), provenant de l'expansion thermique.

2. Détecteur selon une quelconque revendication précédente, dans lequel deux zones (29) qui sont conductrices de chaleur ou de courant sont prévues sur le substrat (6), et deux conducteurs (18) dépassent du corps pour contacter les zones respectives (29) du substrat (6), pour transmettre de la chaleur ou du courant des zones (29) aux couches ou revêtements réfléchissants formés sur le couvercle (13) et la base (1).

3. Détecteur selon la revendication 1 ou 2, dans lequel la connexion comprend une ou plusieurs quantités de matériau conducteur flexible.

4. Détecteur selon la revendication 3, dans lequel le matériau conducteur flexible est formé à partir d'un matériau non conducteur présentant une pluralité de grains ou fibres d'un matériau conducteur enrobés dans celui-ci ou revêtus sur une surface de celui-ci.

5. Détecteur selon la revendication 3 ou 4, dans lequel la connexion comprend une ou plusieurs bandes (18) du matériau conducteur flexible.

6. Détecteur selon l'une quelconque des revendications 3 à 5, dans lequel le matériau conducteur flexible comprend un matériau polymère présentant une pluralité de grains ou fibres d'un métal enrobés dans celui-ci ou revêtus sur une surface de celui-ci.

7. Détecteur selon une quelconque revendication précédente, dans lequel le conducteur (18) est prévu sur une extension (28) du couvercle (13) qui dépasse vers le substrat (6).

8. Détecteur selon une quelconque revendication précédente, comprenant en outre une source et un détecteur, la source émettant un rayonnement qui est réfléchi par les couches ou revêtements réfléchissants formés sur les surfaces intérieures (16, 4) du couvercle (13) et de la base (1) avant d'être absorbé par un détecteur.

9. Détecteur selon une quelconque revendication précédente, dans lequel le couvercle (13) et la base (1) sont formés à partir d'un ou de plusieurs matériaux présentant une faible conductibilité électrique.
